# EUROPEAN PATENT SPECIFICATION

(11) **EP 1 794 248 B1**
(45) Date of publication and mention of the grant of the patent: **27.07.2011**
(21) Application number: 05808399.9
(22) Date of filing: 15.09.2005
(51) Int. Cl.: C08J 5/12

(54) **ELASTOMERIC RADIOPAQUE ADHESIVE COMPOSITE AND PROSTHESIS**
ELASTOMERE UND RÖNTGENDICHTE HAFTZUSAMMENSETZUNG UND PROTHESE
COMPOSITE ADHÉSIF RADIO-OPAQUE ÉLASTOMÈRE ET PROTHÈSE

(30) Priority: 15.09.2004 US 941189
(43) Date of publication of application: 13.06.2007
(73) Proprietor: Boston Scientific Limited, Christ Church (BB)
(72) Inventor: THISTLE, Robert, C., Bridgewater, MA 02324 (US); DIMATTEO, Kristian, Waltham, MA 02451 (US)
(74) Representative: Intès, Didier Gérard André
(86) International application number: PCT/US2005/032865
(87) International publication number: WO 2006/041607

(56) References cited:
- EP-A- 0 399 140
- EP-A- 0 409 427
- GB-A- 1 412 970
- GB-A- 2 169 906
- US-A- 4 645 490
- US-A- 5 106 301
- US-A- 5 645 532
- US-A1- 2004 122 509
- US-B1- 6 296 604

## Description

### FIELD OF THE INVENTION:

The present invention relates generally to an elastomeric radiopaque adhesive composition. More particularly, the present invention relates to a composition including a biocompatible elastomeric matrix and radiopaque material distributed therein. Further, the elastomeric radiopaque adhesive may be used in a prosthesis. The prosthesis includes a textile layer, a polytetrafluoroethylene layer (PTFE) and a cured elastomeric bonding agent layer having radiopaque material impregnated within the PTFE porous layer, which joins the textile and PTFE layer to form an integral structure.

### BACKGROUND OF THE INVENTION:

Implantable prostheses are commonly used in medical applications. One of the more common prosthetic structures is a tubular prosthesis which may be used as a vascular graft to replace or repair damaged or diseased blood vessel. The prostheses may be used to prevent or treat a wide variety of defects such as stenosis of the vessel, thrombosis, occlusion, dissection or an aneurysm. To maximize the effectiveness of such a prosthesis, it should be designed with characteristics which closely resemble that of the natural body lumen which it is repairing or replacing.

A known example of prosthesis used as a vascular graft is disclosed in patent document US 2004/122 509 A1.

One type of implantable prosthesis used in the repair of diseases in various body vessels is a stent. A stent is a generally longitudinal tubular device formed of biocompatible material which is useful to open and support various lumens in the body. For example, stents may be used in the vascular system, urogenital tract, tracheal/bronchial tubes and bile duct, as well as in a variety of other applications in the body. Endovascular stents have become widely used for the treatment of stenosis, strictures and aneurysms in various blood vessels. These devices are implanted within the vessel to open and/or reinforce collapsing or partially occluded sections of the vessel.

Stents generally include an open flexible configuration. This configuration allows the stent to be inserted through curved vessels. Furthermore, this configuration allows the stent to be configured in a radially compressed state for intraluminal catheter implantation. Once properly positioned adjacent the damaged vessel, the stent is radially expanded so as to support and reinforce the vessel. Radial expansion of the stent may be accomplished by inflation of a balloon attached to the catheter or the stent may be of the self expanding variety which will radially expand once deployed.

A graft is another commonly known type of implantable prosthesis which is used to repair and replace various body vessels. A graft provides a lumen through which blood may flow. Moreover, a graft is often configured to have porosity to permit the ingrowth of cells for stabilization of an implanted graft while also being generally impermeable to blood to inhibit substantial leakage of blood therethrough. Grafts are typically tubular devices which may be formed of a variety of materials, including textile and non-textile materials.

Grafts may be flexible to provide compliance within a bodily lumen or within the bodily system. Such flexibility may result from the stretching of the textile yarns that form the graft. Such stretching, however, may effect the securement of the graft to the bodily lumen, which is typically secured by the use of sutures. In other words, the graft flexibility may create undesirable stresses at the suture locations of the implanted graft.

A stent and a graft may be combined to form an implantable prosthesis, such as a stent-graft, which may dilate over time after implantation within a bodily lumen. The dilation of the implanted prosthesis is a radial enlargement of the device resulting from pulsating stresses or pressures present within the bodily lumen. The actions of the pulsating stresses or pressures often fatigue the structure of the device resulting in radial expansion and possibly longitudinal foreshortening.

One form of a conventional tubular prosthesis specifically used for vascular grafts includes a textile tubular structure formed by weaving, knitting, braiding or any non-woven textile technique processing synthetic fibers into a tubular configuration. Tubular textile structures have the advantage of being naturally porous which allows desired tissue ingrowth and assimilation into the body. This porosity, which allows for ingrowth of surrounding tissue, must be balanced with fluid tightness so as to minimize leakage during the initial implantation stage.

Attempts to control the porosity of the graft while providing a sufficient fluid barrier have focused on increasing the thickness of the textile structure, providing a tighter stitch construction and incorporating features such as velours to the graft structure. Further, most textile grafts require the application of a biodegradable natural coating, such as collagen or gelatin in order to render the graft blood tight. While grafts formed in this manner overcome certain disadvantages inherent in attempts to balance porosity and fluid tightness, these textile prostheses may exhibit certain undesirable characteristics. These characteristics may include an undesirable increase in the thickness of the tubular structure, which makes implantation more difficult. These textile tubes may also be subject to kinking, bending, twisting or collapsing during handling. Moreover, application of a coating may render the grafts less desirable to handle from a tactility point of view.

It is also well known to form a prosthesis, especially a tubular graft, from polymers such as polytetrafluoroethylene (PTFE). A tubular graft may be formed by stretching and expanding PTFE into a structure referred to as expanded polytetrafluoroethylene (ePTFE). Tubes formed of ePTFE exhibit certain beneficial properties as compared with textile prostheses. The expanded PTFE tube has a unique structure defined by nodes interconnected by fibrils. The node and fibril structure defines micropores which facilitate a desired degree of tissue ingrowth while remaining substantially fluid-tight. Tubes of ePTFE may be formed to be exceptionally thin and yet exhibit the requisite strength necessary to serve in the repair or replacement of a body lumen. The thinness of the ePTFE tube facilitates ease of implantation and deployment with minimal adverse impact on the body.

While exhibiting certain superior attributes, ePTFE tubes are not without certain disadvantages. Grafts formed of ePTFE tend to be relatively non-compliant as compared with textile grafts and natural vessels. Further, while exhibiting a high degree of tensile strength, ePTFE grafts are susceptible to tearing. Additionally, ePTFE grafts lack the suture compliance of coated textile grafts. This may cause undesirable bleeding at the suture hole. Thus, the ePTFE grafts lack many of the advantageous properties of certain textile grafts.

It is also known that it is extremely difficult to join PTFE and ePTFE to other materials via adhesives or bonding agents due to its chemically inert and non-wetting character. Wetting of the surface by the adhesive is necessary to achieve adhesive bonding, and PTFE and ePTFE are extremely difficult to wet without destroying the chemical properties of the polymer. Thus, heretofore, attempts to bond ePTFE to other dissimilar materials such as textiles, have been difficult.

Further, endovascular implantation of a graft or stent-graft into the vasculature of a patient involves very precise techniques. Generally, the device is guided to the diseased or damaged portion of a blood vessel via an implantation apparatus that deploys the graft or stent-graft at the desired location. In order to pinpoint the location during deployment, the medical specialist will generally utilize a fluoroscope to observe the deployment by means of x-rays. Deployment of a prosthesis at an unintended location can result in medical trauma, as well as increasing the invasiveness associated with multiple deployment attempts and/or relocation of a deployed device. In addition, visualization of the implanted device is essential for follow-up inspection and treatment. However, in order to implant the prosthesis using fluoroscopy, some portion of the prosthesis must be radiopaque. Therefore, there exists a need to provide a radiopaque marker for incorporation into an implantable device which allows the device to contract and expand without interference upon delivery and deployment within the blood vessel of a patient.

It is apparent that conventional textile prostheses as well as ePTFE prostheses have acknowledged advantages and disadvantages. Neither of the conventional prosthetic materials exhibits fully all of the benefits desirable for use as a vascular prosthesis.

It is therefore desirable to provide an elastomeric radiopaque adhesive composition and an implantable prosthesis including the composition, which achieves many of the above-stated benefits without the resultant disadvantages associated therewith.

Known examples of radiopaque adhesive composition are disclosed in patent documents US 5 106 301 A, GB 2169 906 A and US 5 645 532 A.

### SUMMARY OF THE INVENTION:

The present disclosure provides an elastomeric radiopaque adhesive composition which may be used in various applications, especially vascular applications. The elastomeric radiopaque adhesive composition of the present disclosure may include a biocompatible elastomeric matrix and a radiopaque material distributed therein in sufficient amounts to produce a radiopaque image.

An embodiment of the present invention includes a hybrid prosthesis. The prosthesis of the present invention may include a composite structure, a patch, or tubular structure including a textile structure, a polytetrafluoroethylene (PTFE) structure, and a cured elastomeric bonding agent adhesively securing the PTFE structure to the textile structure. The elastomeric agent has radiopaque material impregnated therein in sufficient amounts to produce a radiopaque image. Moreover, additional ePTFE, textile layers and/or stents may be combined with any of these embodiments.

A further embodiment of the present invention is a hybrid vascular prosthesis which includes a textile tubular structure and a PTFE tubular structure. An elastomeric bonding agent having the radiopaque material therein is applied to either the textile structure or the PTFE structure for securing the textile structure to the PTFE structure.

The bonding agent may be selected from a group of materials including biocompatible elastomeric materials such as urethanes, silicones, isobutylene/styrene copolymers, block polymers and combinations thereof. The radiopaque material impregnated therein may include gold, barium sulfate, materials having similar properties and combinations thereof.

The tubular composite grafts of the present invention may also be formed from appropriately layered sheets which can then be overlapped to form tubular structures. Bifurcated, tapered conical and stepped-diameter tubular structures may also be formed from the present invention.

The textile structure includes knits, weaves, stretch knits, braids, any non-woven textile processing techniques, and combinations thereof. Various biocompatible polymeric materials may be used to form the textile structures, including polyesters, polyethylene terephthalate (PET), naphthalene dicarboxylate derivatives such as polyethylene naphthalate, polybutylene naphthalate, polytrimethylene naphthalate, trimethylenediol naphthalate, polytetrafluoroethylene (PTFE), ePTFE, natural silk, polyethylene and polypropylene, among others.

The radiopaque bonding agent may be applied in a number of different manners to either the textile layer or the PTFE layer. Preferably, the radiopaque bonding agent is applied in solution to one surface of the PTFE layer, preferably by spray coating. The textile layer is then placed in contact with the coated surface of the PTFE layer. The radiopaque bonding agent may also alternatively be in the form of a solid tubular structure. The radiopaque bonding agent may also be applied in powder form, and may also be applied and activated by thermal and/or chemical processing well known in the art.

The present invention further provides a hybrid vascular prosthesis including a PTFE tubular structure, a textile tubular structure, a support tubular structure, and a cured elastomeric bonding agent adhesively securing the PTFE structure, textile structure and support structure. The elastomeric agent is impregnated with radiopaque material in sufficient amounts to produce a radiopaque image.

The present invention, more specifically provides for a hybrid composite endovascular prosthesis. The prosthesis includes a braided stent frame, a nonporous PTFE layer covering the stent frame, a polyester knitted textile layer covering the PTFE layer, and a cured radiopaque elastomeric composition thermally bonding the PTFE layer and the textile layer. The radiopaque elastomeric composition includes radiopaque metallic particulates and polyurethane adhesive. The polyurethane adhesive is impregnated with the particulates in sufficient amounts to produce a radiopaque image. Additionally, this embodiment may not contain a stent, or may contain additional layers of textile and/or PTFE. Furthermore, the arrangement may vary, in that, the stent may be placed between the PTFE and textile layer, into the elastomeric layer; the PTFE-elastomer-textile composite may be placed and attached within the stent structure; or the textile and PTFE layers may be inverted such that the textile is the inner layer and the PTFE is exterior. Various combinations may be contemplated herein with the scope of the invention.

Furthermore, the present invention provides an implantable patch which may be used to cover an incision made in a blood vessel, or otherwise support or repair a soft tissue body part, such as a vascular wall or muscular tissue, i.e. hernia repair patch. The patch of the present invention includes a PTFE substantially planar structure, a textile substantially planar structure, and a cured elastomeric bonding agent therebetween adhesively securing the PTFE structure and textile structure thereto. The elastomeric agent has radiopaque material impregnated therein is sufficient amounts to produce a radiopaque image.

Additionally, provided is a method of forming a hybrid device of the present invention. The method includes the steps of providing a PTFE layer having opposed surfaces, providing a textile layer having opposed surfaces and providing a radiopaque elastomeric composition including an elastomeric bonding agent and radiopaque metallic particulates. The particulates are impregnated into the elastomeric agent in sufficient amounts to produce a radiopaque image upon implantation of the device. A layer of said radiopaque elastomeric composition is applied to one of the opposed surfaces of the PTFE layer or textile layer. The opposed surface of the other of said PTFE layer or textile layer is placed on top of said layer of radiopaque elastomeric composition thereby defining a hybrid assembly. The hybrid assembly has an interior surface and exterior surface, and the interior surface being one of the PTFE layer is textile layer and the exterior surface being the other of the PTFE layer or the textile layer. Heat is applied to the radiopaque elastomeric composition to adhesively bond the textile layer and the PTFE layer, and the radiopaque material is encapsulated between the textile layer and PTFE layer to provide a laminated hybrid assembly.

The composite multi-layered implantable structures of the present invention are designed to take advantage of the inherent beneficial properties of the materials forming each of the layers. The textile layer provides for enhanced tissue ingrowth, high suture retention strength and longitudinal compliance for ease of implantation. The PTFE layer provides the beneficial properties of sealing the textile layer without need for coating the textile layer with a sealant such as collagen. The sealing properties of the PTFE layer allow the wall thickness of the textile layer to be minimized. Further, the PTFE layer exhibits enhanced thrombo-resistance upon implantation. Moreover, the elastomeric bonding agent not only provides for an integral composite structure, but also may add further puncture-sealing characteristics to the final prosthesis. The radiopaque material provides a way to monitor the delivery, deployment, and continued monitoring of the inventive endovascular prosthesis. Additionally, the radiopaque material provides a way to continually monitor the inventive surgically implanted devices, such as a surgical graft.

It is well within the contemplation of the present invention that the elastomeric radiopaque adhesive composition or the inventive prosthesis, or layers thereof can be combined with various carrier, drug, prognostic, or therapeutic materials. For example, the elastomeric radiopaque adhesive composition, PTFE layer, or textile layer can be combined or coated with any of the following therapeutic agents: antimicrobial agents, such as the antibiotic agents and antiseptic agents listed above; anti-thrombogenic agents, such as heparin, heparin derivatives, urokinase, and PPack (dextrophenylalanine proline, arginine, chloromethylketone); anti-proliferative agents (such as enoxaprin, angiopeptin, or monoclonal antibodies capable of blocking smooth muscle cell proliferation, hirudin, and acetylsalicylic acid); anti-inflammatory agents, such as dexamethasone, prednisolone, corticosterone, budesonide, estrogen, sulfasalazine, and mesalamine); anti-neoplastics/anti-proliferative/anti-miotic agents (such as paclitaxel, 5-flurouracil, cisplatin, vinblastine, vincristine, epothilones, endostatin, angiostatin and thymidine kinase inhibitors); anesthetic agents (such as lidocaine, bupivacaine, and ropivacaine); anti-coagulants (such as D-Phe-Pro-Arg chloromethyl keton, an RGD peptide-containing compound, heparin, antithrombin compounds, platelet receptor antagonists, anti-thrombin antibodies, anti-platelet receptor antibodies, aspirin, prostaglandin inhibitors, platelet inhibitors and tick anti-platelet peptides); vascular cell growth promoters (such as growth factor inhibitors, growth factor receptor antagonists, transcriptional activators, and translational promoters); vascular cell growth inhibitors (such as growth factor inhibitors, growth factor receptor antagonists, transcriptional repressors, translational repressors, replication inhibitors, inhibitory antibodies, antibodies directed against growth factors, bi-functional molecules consisting of a growth factor and a cytotoxin, bi-functional molecules consisting of an antibody and a cytotoxin); cholesterol-lowering agents; vasodilating agents; and agents which interfere with andogenous or vascoactive mechanisms. In addition, cells which are able to survive within the body and are dispersed within the coating layer may be therapeutically useful. These cells themselves may be therapeutically useful or they may be selected or engineered to produce and release therapeutically useful compositions.

### BRIEF DESCRIPTION OF THE DRAWINGS:

Figure 1 shows a schematic view of a cured elastomeric radiopaque adhesive composition including an elastomeric matrix and radiopaque material distributed therein of the present invention.
Figure 2 shows a schematic cross-section of a portion of a composite multi-layered implantable structure having radiopaque material uniformally distributed throughout the elastomeric matrix of the present invention.
Figure 2A shows a perspective view of a prosthesis of the present invention having an internal layer of PTFE structure and an external layer of textile structure.
Figure 2B shows a cross-sectional view of the prosthesis of Figure 2A of the present invention.
Figure 3 shows a schematic view of a portion of a composite multi-layered implantable structure having radiopaque material concentrated in designated areas with the elastomeric matrix of the present invention.
Figure 4 shows a schematic view of a portion of a composite multi-layered implantable structure having radiopaque material uniformally distributed throughout the elastomeric matrix of the present invention.
Figure 4A shows a perspective view of a prosthesis of the present invention having an internal layer of textile structure and an external layer of PTFE structure.
Figure 4B shows a cross-sectional view of the prosthesis of Figure 4A of the present invention.
Figure 5 shows a perspective view of a composite prosthesis of the present invention including a stent covered by PTFE-textile composite.
Figure 5A shows a cross-sectional view of the prosthesis of Figure 5 of the present invention.
Figure 6 show a perspective view of a composite prosthesis and a stent incorporated therein of the present invention.
Figure 6A shows a cross-sectional view of the prosthesis of Figure 6 of the present invention.
Figure 7 shows a perspective view of a composite PTFE-textile vascular patch of the present invention.
Figure 7A shows a cross-sectional view of the prosthesis of Figure 7 of the present invention.

### DETAILED DESCRIPTION OF THE PREFERRED EMBODIMENT:

The present invention provides for an elastomeric radiopaque adhesive composition. The composition includes a biocompatible elastomeric matrix and a radiopaque material distributed therein in sufficient amounts to produce a radiopaque image.

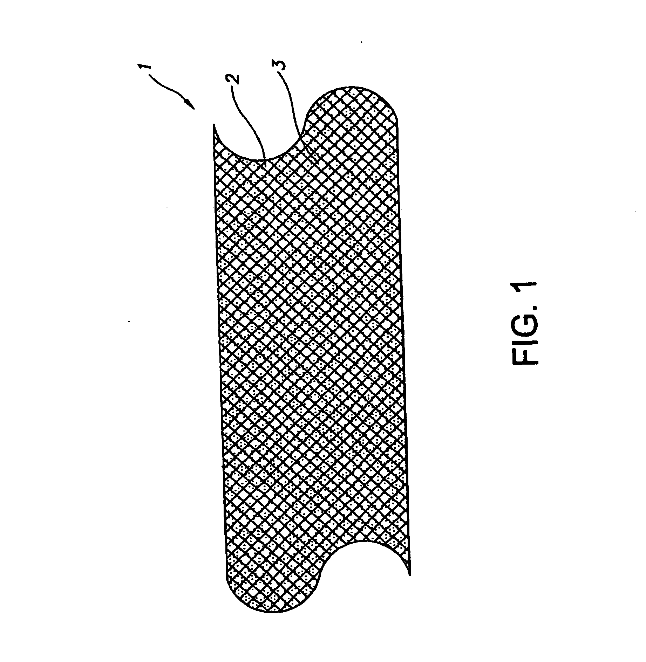

Referring to Figure 1, a schematic view of a portion of a cured elastomeric radiopaque adhesive composition 1 of the present invention is shown. The composition 1 may be used and formed into various devices for implantation use such as a graft, patch, prosthesis or any other implantable structure, or portion thereof.

The composition 1 includes a biocompatible elastomeric matrix **2** and a radiopaque material 3 distributed therein in sufficient amounts to produce a radiopaque image. Preferably, sufficient amounts of radiopaque material is provided to produce a radiopaque image in an implanted situation.

In the present invention, the elastomeric matrix **2** may include various biocompatible, elastomeric bonding agents such as urethanes, styrene/isobutylene/styrene block copolymers (SIBS), silicones, and combinations thereof. Other similar materials are contemplated. Most desirably, the bonding agent may include polycarbonate urethanes sold under the trade name CORETHANE^{®}. This urethane is provided as an adhesive solution with preferably 7.5% Corethane, 2.5 W30, in dimethylacetamide (DMAc) solvent.

The term elastomeric as used herein refers to a substance having the characteristic that it tends to resume an original shape after any deformation thereto, such as stretching, expanding or compression. It also refers to a substance which has a non-rigid structure, or flexible characteristics in that it is not brittle, but rather has compliant characteristics contributing to its non-rigid nature.

The polycarbonate urethane polymers particularly useful in the present invention are more fully described in U.S. Patent Nos. 5,133,742 and 5,229,431 . These polymers are particularly resistant to degradation in the body over time and exhibit exceptional resistance to cracking *in vivo*. These polymers are segmented polyurethanes which employ a combination of hard and soft segments to achieve their durability, biostability, flexibility and elastomeric properties.

The polycarbonate urethanes useful in the present invention are prepared from the reaction of an aliphatic or aromatic polycarbonate macroglycol and a diisocyanate n the presence of a chain extender. Aliphatic polycarbonate macroglycols such as polyhexane carbonate macroglycols and aromatic diisocyanates such as methylene diisocyanate are most desired due to the increased biostability, higher intramolecular bond strength, better heat stability and flex fatigue life, as compared to other materials.

The polycarbonate urethanes particularly useful in the present invention are the reaction products of a macroglycol, a diisocyanate and a chain extender.

A polycarbonate component is characterized by repeating 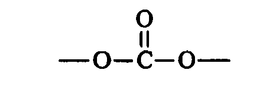 units, and a general formula for a polycarbonate macroglycol is as follows: 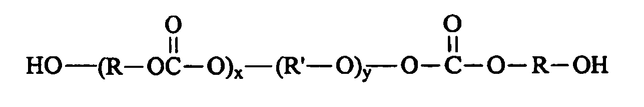 wherein x is from 2 to 35, y is 0, 1 or 2, R either is cycloaliphatic, aromatic or aliphatic having from about 4 to about 40 carbon atoms or is alkoxy having from about 2 to about 20 carbon atoms, and wherein R has from about 2 to about 4 linear carbon atoms with or without additional pendant carbon groups.

Examples of typical aromatic polycarbonate macro glycols include those derived from phosgene and bisphenol A or by ester exchange between bisphenol A and diphenyl carbonate such as (4,4-dihydroxy-diphenyl-2,2-propane) shown below, wherein n is between about 1 and about 12.

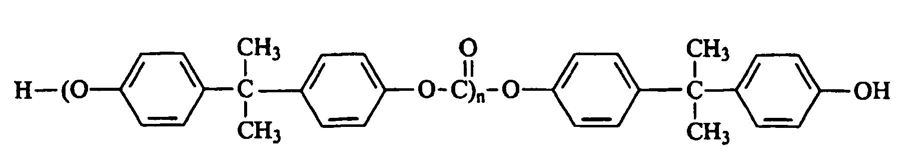

Typical aliphatic polycarbonates are formed by reacting cycloaliphatic or aliphatic diols with alkylene carbonates as shown by the general reaction below: 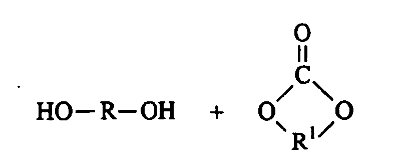 wherein R is cyclic or linear and has between about 1 and about 40 carbon atoms and wherein R¹ is linear and has between about 1 and about 4 carbon atoms.

Typical examples of aliphatic polycarbonate diols include the reaction products of 1,6-hexanediol with ethylene carbonate, 1,4-butanediol with propylene carbonate, 1,5-pentanediol with ethylene carbonate, cyclohexanedimethanol with ethylene carbonate and the like and mixtures of above such as diethyleneglycol and cyclohexanedimethanol with ethylene carbonate.

When desired, polycarbonates such as these can be copolymerized with components such as hindered polyesters, for example phthalic acid, in order to form carbonate/ester copolymer macroglycols. Copolymers formed in this manner can be entirely aliphatic, entirely aromatic, or mixed aliphatic and aromatic. The polycarbonate macroglycols typically have a molecular weight of between about 200 and about 4000 Daltons.

Diisocyanate reactants according to this invention have the general structure OCN-R - NCO, wherein R' is a hydrocarbon that may include aromatic or nonaromatic structures, including aliphatic and cycloaliphatic structures. Exemplary isocyanates include the preferred methylene diisocyanate (MDI), or 4,4-methylene bisphenyl isocyanate, or 4,4'-diphenylmethane diisocyanate and hydrogenated methylene diisocyanate (HMDI). Other exemplary isocyanates include hexamethylene diisocyanate and other toluene diisocyanates such as 2,4-toluene diisocyanate and 2,6-toluene diisocyanate, 4,4' tolidine diisocyanate, m-phenylene diisocyanate, 4-chloro-1,3-phenylene diisocyanate, 4,4-tetramethylene diisocyanate, 1,6-hexamethylene diisocyanate, 1,10-decamethylene diisocyanate, 1,4-cyclohexylene diisocyanate, 4,4'-methylene bis (cyclohexylisocyanate), 1,4-isophorone diisocyanate, 3,3'-dimethyl-4,4'-diphenylmethane diisocyanate, 1,5-tetrahydronaphthalene diisocyanate, and mixtures of such diisocyanates. Also included among the isocyanates applicable to this invention are specialty isocyanates containing sulfonated groups for improved hemocompatibility and the like.

Suitable chain extenders included in this polymerization of the polycarbonate urethanes should have a functionality that is equal to or greater than two. A preferred and well-recognized chain extender is 1,4-butanediol. Generally speaking, most diols or diamines are suitable, including the ethylenediols, the propylenediols, ethylenediamine, 1,4-butanediamine methylene dianiline heteromolecules such as ethanolamine, reaction products of said diisocyanates with water and combinations of the above.

The polycarbonate urethane polymers according to the present invention should be substantially devoid of any significant ether linkages (i.e., when y is 0, 1 or 2 as represented in the general formula hereinabove for a polycarbonate macroglycol), and it is believed that ether linkages should not be present at levels in excess of impurity or side reaction concentrations. While not wishing to be bound by any specific theory, it is presently believed that ether linkages account for much of the degradation that is experienced by polymers not in accordance with the present invention due to enzymes that are typically encountered in vivo, or otherwise, attack the ether linkage via oxidation. Live cells probably catalyze degradation of polymers containing linkages. The polycarbonate urethanes useful in the present invention avoid this problem.

Because minimal quantities of ether linkages are unavoidable in the polycarbonate producing reaction, and because these ether linkages are suspect in the biodegradation of polyurethanes, the quantity of macroglycol should be minimized to thereby reduce the number of ether linkages in the polycarbonate urethane. In order to maintain the total number of equivalents of hydroxyl terminal groups approximately equal to the total number of equivalents of isocyanate terminal groups, minimizing the polycarbonate soft segment necessitates proportionally increasing the chain extender hard segment in the three component polyurethane system. Therefore, the ratio of equivalents of chain extender to macroglycol should be as high as possible. A consequence of increasing this ratio (i.e., increasing the amount of chain extender with respect to macroglycol) is an increase in hardness of the polyurethane. Typically, polycarbonate urethanes of hardnesses, measured on the Shore scale, less than 70A show small amounts of biodegradation. Polycarbonate urethanes of Shore 75A and greater show virtually no biodegradation.

The ratio of equivalents of chain extender to polycarbonate and the resultant hardness is a complex function that includes the chemical nature of the components of the urethane system and their relative proportions. However, in general, the hardness is a function of the molecular weight of both chain extender segment and polycarbonate segment and the ratio of equivalents thereof. Typically, the 4,4'-methylene bisphenyl diisocyanate (MDI) based systems, a 1,4-butanediol chain extender of molecular weight 90 and a polycarbonate urethane of molecular weight of approximately 2000 will require a ratio of equivalents of at least about 1.5 to 1 and no greater than about 12 to 1 to provide non-biodegrading polymers. Preferably, the ratio should be at least about 2 to 1 and less than about 6 to 1. For a similar system using a polycarbonate glycol segment of molecular weight of about 1000, the preferred ration should be at least about 1 to 1 and no greater than about 3 to 1. A polycarbonate glycol having a molecular weight of about 500 would require a ratio in the range of about 1.2 to about 1.5:1.

The lower range of the preferred ratio of chain extender to macroglycol typically yields polyurethanes of Shore 80A hardness. The upper range of ratios typically yields polycarbonate urethanes on the order of Shore 75D. The preferred elastomeric and biostable polycarbonate urethanes for most medical devices would have a Shore hardness of approximately 85A.

Generally speaking, it is desirable to control somewhat the cross-linking that occurs during polymerization of the polycarbonate urethane polymer. A polymerized molecular weight of between about 80,000 and about 200,000 Daltons, for example on the order of about 120,000 Daltons (such molecular weights being determined by measurement according to the polystyrene standard), is desired so that the resultant polymer will have a viscosity at a solids content of 43% of between about 900,000 and about 1,800,000 centipoise, typically on the order of about 1,000,000 centipoise. Cross-linking can be controlled by avoiding an isocyanate-rich situation. Of course, the general relationship between the isocyanate groups and the total hydroxyl (and/or amine) groups of the reactants should be on the order of approximately 1 to 1. Cross-linking can be controlled by controlling the reaction temperatures and shading the molar ratios in a direction to be certain that the reactant charge is not isocyanate-rich; alternatively a termination reactant such as ethanol can be included in order to block excess isocyanate groups which could result in cross-linking which is greater than desired.

Concerning the preparation of the polycarbonate urethane polymers, they can be reacted in a single-stage reactant charge, or they can be reacted in multiple states, preferably in two stages, with or without a catalyst and heat. Other components such as antioxidants, extrusion agents and the like can be included, although typically there would be a tendency and preference to exclude such additional components when a medical-grade polymer is being prepared.

Additionally, the polycarbonate urethane polymers can be polymerized in suitable solvents, typically polar organic solvents in order to ensure a complete and homogeneous reaction. Solvents include dimethylacetamide, dimethylformamide, dimethylsulfoxide toluene, xylene, m-pyrrol, tetrahydrofuran, cyclohexanone, 2-pyrrolidone, and the like, or combinations thereof.

A particularly desirable polycarbonate urethane is the reaction product of polyhexamethylenecarbonate diol, with methylene bisphenyl diisocyanate and the chain extender 1,4-butanediol.

In a preferred embodiment, the elastomeric matrix may contribute to re-sealable qualities, or puncture-sealing characteristics of the composite structure. If the bonding agent is a highly elastic substance, this may impart re-sealable quantities to the composite structure. This is especially desirous in order to seal a hole created by a suture, or when the self-sealing graft may be preferably used as a vascular access device. When used as an access device, the graft allows repeated access to the blood stream through punctures, which close after removal of the penetrating member (such as, e.g., a hypodermic needle or cannula) which provided the access.

In the present invention, the elastomeric matrix **2** includes radiopaque material **3** impregnated therein in sufficient amounts to produce a radiopaque image. The radiopaque material **3** is a substance which is biocompatible, provides a radiopaque image, and is not necessarily MRI (magnetic resonance imaging) safe. Suitable radiopaque material may include various materials as known in the art which provide radiopaque images, such as metalloids, metallic material, gold, barium sulfate, ferritic particles, platinum, platinum-tungsten, palladium, platinum-iridium rhodium, tantalum or alloys or composites and the like, including various combinations thereof.

The radiopaque material 3 may be present in various forms depending on the end use. For example, the material may be present in the form of shavings, slivers, filings, droplets, flakes, powder, particles or particulates ranging between about 0.2 microns to about 3.0 microns. More preferably, the size range of the particles is between about 0.5 microns to about 2.0 microns.

The elastomeric matrix **2** includes enough radiopaque material **3** to provide radiopaque imagery upon implantation of the radiopaque elastomeric adhesive composition 1. Therefore, the elastomeric matrix **2** may include between about 1% to about 50% radiopaque material, preferably between about 5% and about 40% radiopaque material therein.

Additionally, the distribution and orientation of the radiopaque material within the elastomeric matrix may vary depending on the desired use. For example, the radiopaque material may be uniformally distributed throughout the elastomeric matrix or concentrated in designated areas. Further, a pattern may be created within the elastomeric matrix, such as hatch marks, gradient indicators, measurable scale and the like. The radiopaque material may be used as a tool to assist doctors by providing a visual image to monitor a prosthesis for degradation, tears, wear, improper implantation, movement, and the like, or by providing sizing, concentration, or manufacturing detailed information of the prosthesis implanted therein.

Radiopaque material 3 may be combined with an elastomeric material by various methods, depending on the desired end use, to provide the elastomeric radiopaque adhesive composition. In addition to the desire end product, the particular process conditions employed and specific materials used dictate the preferred method of forming the elastomeric radiopaque adhesive composition. For example, the radiopaque material may be desired to be presented in a pattern within the elastomeric matrix. One method includes applying the radiopaque material in the desired pattern on top of the uncured, or semi-cured elastomeric material. Depending on the thickness of the elastomeric material and state-of-cure, the radiopaque material may settle therein, or a top coat of elastomeric material may be applied to seal the radiopaque material therein. Alternatively, a homogeneous elastomeric matrix may be desired. This may be accomplished by mixing the radiopaque material and the elastomeric material together while both are in a dry, solid state. The mixture is then slowly introduced to a solvent, under constant mixing, and heat is added, if needed. The solid particles are dissolved and/or dispersed accordingly. As the elastomer cures, the radiopaque particles suspended therein are locked in place within the elastomeric matrix. Similarly, another method which provides a homogeneous mixture includes preparing the elastomeric material into a solution, and mixing or dissolving the radiopaque material therein. The solution or suspension mixture is continually mixed and heat may be, as needed, until the elastomer cures suspending the radiopaque material therein. A further method of providing a homogeneous mixture includes preparing a solution of elastomeric material, and a solution of radiopaque material, and simultaneously combining the solutions upon a substrate to cure by use of a dual spray head, for example. Various other methods of suspending a particle within a solution are known in the art and may be used to provide the elastomeric matrix of the present invention.

Other embodiments of the present invention provide for implantable prostheses including a layer of PTFE and a layer of a textile material which are secured together by an elastomeric bonding agent having radiopaque material impregnated therein in sufficient amounts to produce a radiopaque image. The prosthesis may be a graft, a patch, or other similar implantable devices.

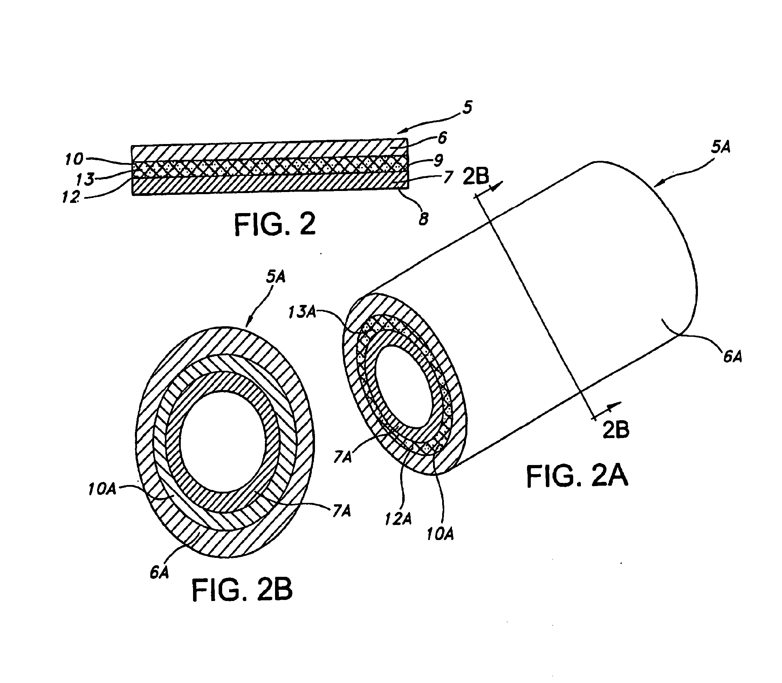

Referring to Figure 2, a schematic cross-section of a portion of a representative prosthesis **5** is shown. As noted above, the prosthesis **5** may be a portion of a graft, patch or any other implantable structure.

The prosthesis **5** includes a first layer or structure **6** which is formed of a textile material. The textile layer **6** of the present invention may be formed from natural or synthetic yarns that provide a textile layer that is biocompatible and biodurable, ie. not biodegradable, within the body lumen. Preferably, the yarns are made from thermoplastic materials including, but not limited to, polyesters, polypropylenes, polyethylenes, polyurethanes, polynaphthalenes, polytetrafluoroethylenes and the like. The yarns may be of the multifilament, monofilament or spun types. In most vascular applications, multifilaments are preferred due to the increase in flexibility. Where enhanced crush resistance is desired, the use of monofilaments have been found to be effective. Additionally, the yarns may be flat, shaped, twisted, textured, pre-shrunk or un-shrunk depending on the desired end use. As is well known, the type and denier of the yam chosen are selected in a manner which forms a durable and pliable soft tissue prosthesis and, more particularly, a vascular structure have desirable properties.

The textile portions of the present invention can have virtually any textile construction, including weaves, knits, braids, filament windings and the like. Useful weaves include, but are not limited to, simple weaves, basket weaves, twill weaves, satin weaves, velour weaves and the like. Useful knits include, but are not limited to high stretch knits, locknit knits (also referred to as tricot or jersey knits), reverse locknit knits, sharkskin knits, queenscord knits and velour knits. Useful high stretch, warp-knitted patterns include those with multiple patterns of diagonally shifting yarns, such as certain modified atlas knits which are described in U.S. Patent No. 6,540,773 . Other useful high-stretch, warp knitted patterns include certain patterns with multiple needle underlap and one needle overlap, such as those patterns described in U.S. Patent No. 6,554,855 and U.S. Patent Application Publication No. 2003/0204241 A1 .

The prosthesis **5** further includes a second layer or structure **7** formed of polytetrafluoroethylene (PTFE). The PTFE layer **7** may be porous or nonporous, or expanded polytetrafluoroethylene (ePTFE). The non-porous PTFE is defined as having a porosity of about 100 ml/min/cm² or less.

The PTFE layer **7,** for example, may be produced from the expansion of PTFE formed in a paste extrusion process. The PTFE extrusion may be expanded and sintered in a manner well known in the art to form ePTFE having a microporous structure defined by nodes interconnected by elongate fibrils. The distance between the nodes, referred to as the internodal distance (IND), may be varied by the parameters employed during the expansion and sintering process. The resulting process of expansion and sintering yields pores within the structure of the ePTFE layer. The size of the pores are defined by the IND of the ePTFE layer.

The composite prosthesis **5** of the present invention further includes a bonding agent **10** which adhesively secures the PTFE layer **7** and the textile layer **6.** The bonding agent **10** is similar to the elastomeric adhesive composition **1** of Figure 1. The bonding agent **10** includes an elastomeric matrix **12** and a radiopaque material **13,** similar to that above-described in reference to elastomeric matrix **2** and a radiopaque material **3** of Figure 1. The radiopaque material **13** may be compounded, dissolved or suspended in the elastomeric matrix **12,** as above-described in reference to elastomeric matrix **2.**

The bonding agent **10** is preferably applied in solution by a spray, brush or dip coating process. However, other processes as known in the art may be employed to apply the bonding agent to the layers **6, 7.** For example, a dual spray head may be employed. The dual spray head device includes dual chambers. One chamber contains the elastomeric material and the other chamber containing the radiopacifier. Depressing the spray head releases the contents in both chambers being the elastomeric material and the radiopaque material, distributing them on the substrate layer, i.e. textile or PTFE. The use of the elastomeric bonding agent **10** in solution is particularly beneficial in that by coating the surface **9** of PTFE layer **7,** the bonding agent solution enters the pores **8** of layer **7** defined by the IND of the PTFE layer. As the PTFE is a highly hydrophobic material, it is difficult to apply a bonding agent directly to the surface thereof. By providing a bonding agent which may be disposed within the micropores of the PTFE structure, enhanced bonding attachment between the bonding agent and the PTFE surface is achieved.

The bonding agents of the present invention, particularly the materials noted above and, more particularly, polycarbonate urethanes, such as those formed from the reaction of aliphatic macroglycols and aromatic or aliphatic diisocyanates, are elastomeric materials which exhibit elastic properties. Conventional PTFE is generally regarded as an inelastic material, i.e., even though it can be further stretched, it has little memory. Therefore, conventional PTFE exhibits a relatively low degree of longitudinal compliance. Also, suture holes placed in conventional PTFE structures do not self-seal, due to the inelasticity of the PTFE material. By applying an elastomeric coating to the PTFE structure, both longitudinal compliance and suture hole sealing are enhanced.

Referring again to Figure 2, textile layer **6** is secured to surface **9** of PTFE layer **7** which has been coated with elastomeric bonding agent **10.** The elastomeric bonding agent **10** is impregnated with radiopaque material **13** in sufficient amounts to produce a radiopaque image. The textile layer **6** is secured by placing it in contact with the bonding agent **10.** The radiopaque material **13** which may have rough or sharp material is impregnated in the elastomer matrix **12.** The bonding agent **10** is located between the PTFE layer **7** and textile layer **6** to provide a substantially smooth inner and outer surface of the prosthesis **5** to prevent abrasion, damage or injury to the tissue surrounding the implanted prosthesis **5** and the blood flowing therethrough. As it will be described in further detail hereinbelow, the process of forming the composite prosthesis **5** can be performed either by mechanical, chemical or thermal techniques or combinations thereof.

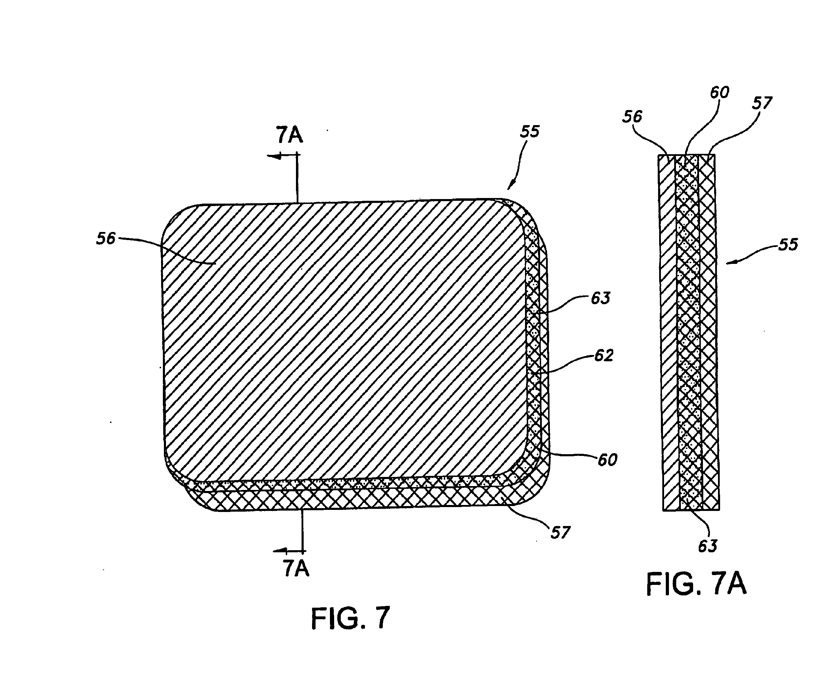

The hybrid prosthesis **5** may be used in various vascular applications in planar form as a vascular patch, as shown in Figure 7, or in tubular form as a graft, as shown in Figure **2A****.**

The hybrid prosthesis **5** may be formed into a tubular graft **5A** as shown in Figure **2A** and 2B. The graft **5A** includes an exterior layer of textile **6A,** and an interior layer of PTFE **7A.** The two layers **6A, 7A** are adhesively secured together by the elastomeric bonding agent **10A** which includes an elastomeric matrix **12A** and a radiopaque material **13A** therein.

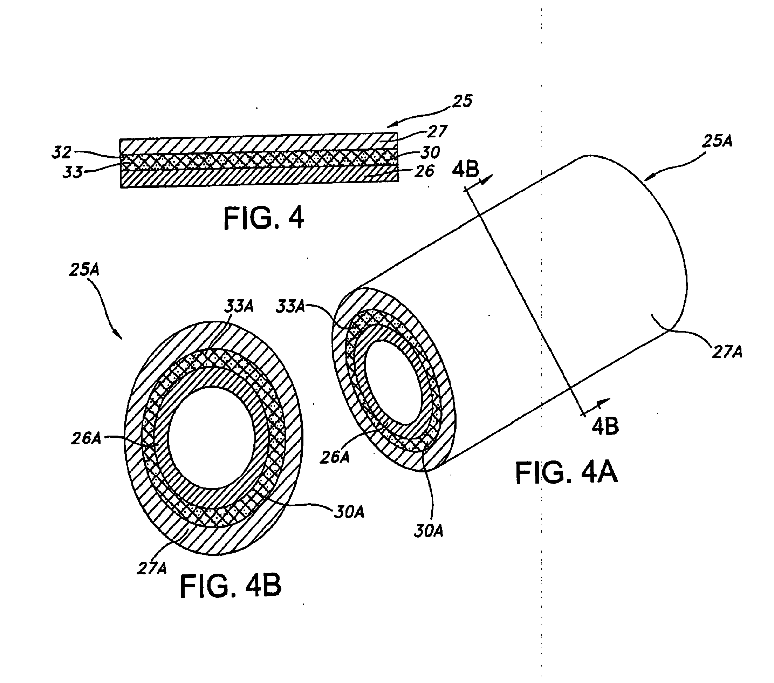

Any implantable device formed from the above-described present invention provides a textile surface which may be designed as a tissue contacting surface in order to promote enhanced cellular ingrowth which contributes to the long term patency of the prosthesis. Further, the PTFE surface may be used as a blood contacting surface so as to minimize leakage and to provide a generally anti-thrombogetic surface. While this is the preferred usage of the composite prosthesis of the present invention, in certain situations, the layers may be reversed where indicated having the textile surface in the interior and the PTFE on the exterior, as shown in Figure 4 and **4A****.**

The radiopaque hybrid graft **5A,** as shown in Figure **2A****,** is desirably formed as follows. A thin PTFE layer **7A** is formed into a tubular structure in conventional forming processes such as by tubular extrusion or by sheet extrusion where the sheet is formed into a tubular configuration. The PTFE tube is placed over a stainless steel mandrel and the ends of the tube are secured. The PTFE tube is then coated with a bonding agent **10A** which includes an elastomeric matrix **12A.** The elastomeric matrix **12A** is made of anywhere from about 1% to about 15% Corethane^{®} urethane range, 2.5 W30 in DMAc. As noted above, other adhesive solutions may also be employed. The bonding agent **10A** also includes radiopaque material **13** encapsulated therein. The PTFE tube is coated with the bonding agent **10A** and if desired, the coating process can be repeated multiple times to add more adhesive to the PTFE tube. The coated PTFE tube is then covered with a textile layer **6A** which was formed into a tube in situ or prior thereto. A textile tube is formed in a conventional forming process such as by braiding, weaving, knitting, non-woven, co-spinning and combinations thereof where the textile is formed into a tubular configuration. The PTFE-radiopaque bonding agent-textile form the composite graft **5A.** One or more layers of elastic tubing, preferably silicone, is then placed over the composite structure. This holds the composite structure together and assures that complete contact and adequate pressure is maintained for bonding purposes. The assembly of the composite graft within the elastic tubing is placed in an oven and heated in a range of about 149°C-260°C (300°F - 500°F) preferably from 163°C (325°F) to 232°C (450°F), for approximately 5-30 minutes to bond the layers together.

The radiopaque PTFE textile graft exhibits advantages over conventional textile grafts in that the PTFE liner acts as a barrier membrane which results in less incidences of bleeding without the need to coat the textile graft in collagen. The wall thickness of the composite structure may be reduced while still maintaining the handling characteristics, especially where the graft may be crimped if desirable. A reduction in suture hole bleeding is seen in that the elastic bonding agent used to bond the textile to the PTFE, renders the PTFE liner self-sealing.

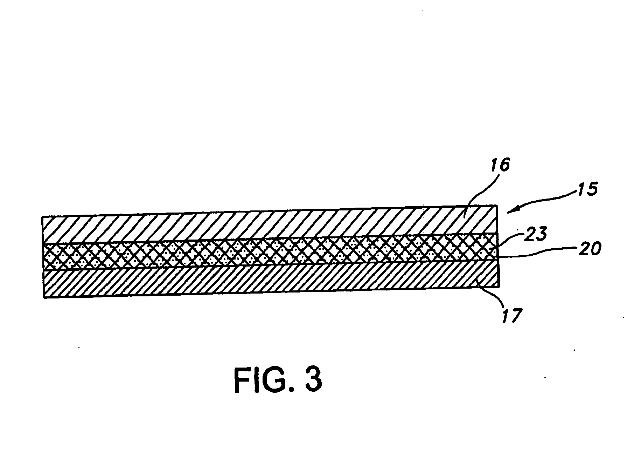

Another embodiment of the present invention is a hybrid composite **15** in Figure 3 which is similar to hybrid prosthesis **5** of Figure 2, having a textile layer or structure **16** and PTFE layer or structure **17** and adhesively securing the structures **16,17** with an elastomeric bonding agent **20** having radiopaque material **23** encapsulated therein. Unlike Figure 2, Figure 3 shows the radiopaque material **23** being concentrated in designated areas, showing a pattern which may be use for a measurable scale, for example.

A further embodiment of the present invention is a composite prosthesis similar to prosthesis **5** of Figure 2 but having the layers inverted as shown in Figure 4. A PTFE covered textile hybrid prosthesis **25** of the present invention includes an PTFE layer **27** having positioned thereover a textile layer **26.** The PTFE layer **27** is bonded to the textile layer **26** by an elastomeric bonding agent **30** including an elastomeric matrix **32** and radiopaque material **33** encapsulated therein. Additionally contemplated is using hybrid prosthesis **25** to form a patch or a tubular graft as shown in Figure **4A****.** As shown in Figures **4A** and **4B****,** hybrid graft **25A** includes a textile layer **26A** on the inner surface and a PTFE layer **27A** covering the textile layer **26A.** The PTFE layer **27A** is provided as the exterior surface of the hybrid graft **25A.** Both layers **26A, 27A** being secured by the elastomeric bonding agent 30A having the radiopaque material **33A** therein. The hybrid graft **25A** of the present invention includes an elongate PTFE tube having positioned thereover a textile tube, as above-described. The process for forming the PTFE covered textile hybrid graft **25A** is similar to that of the textile covered PTFE hybrid graft **5A** as above-described.

Generally, a textile tube is formed in a conventional forming process such as by braiding, weaving, knitting, non-woven, co-spinning and combinations thereof where the textile is formed into a tubular configuration. The textile tube is placed over a stainless steel mandrel and the ends of the tube are secured. The textile tube is then coated with a bonding agent encapsulating radiopaque material therein. The bonding agent may be a variety of elastomeric adhesives and the radiopaque material may vary as previously discussed. If desired, the coating process can be repeated multiple times to add more adhesive to the textile tube. The coated textile tube is then covered with a previously formed PTFE tube, an ePTFE tube, or a PTFE sheet formed into a tube thereon to form the composite prosthesis. The assembly is placed in an oven and heated in the range of about 149°C-260°C (300°F to about 500°F) preferably from 163°C (325°F) to 232°C (450°F) for approximately 5 to 30 minutes depending on the thickness of the adhesive layer, to bond the layers together.

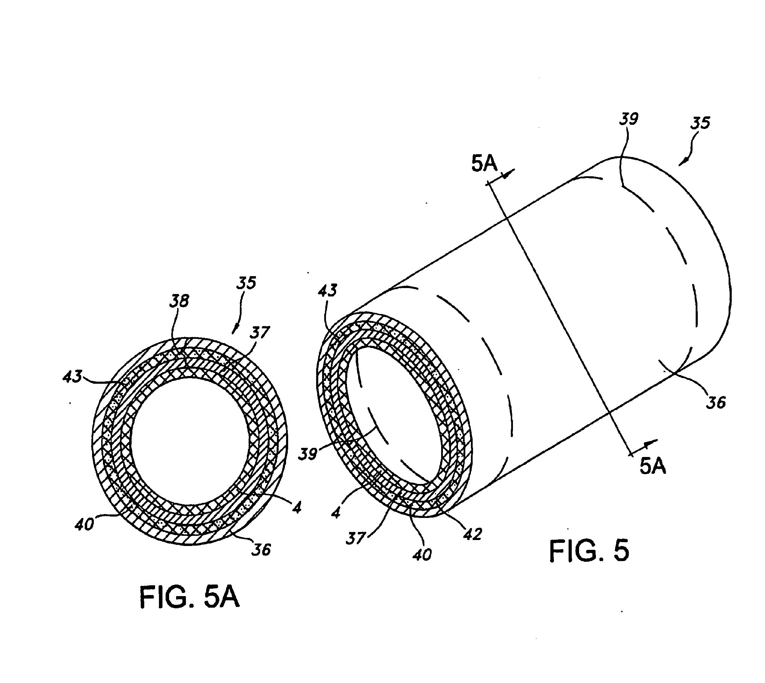

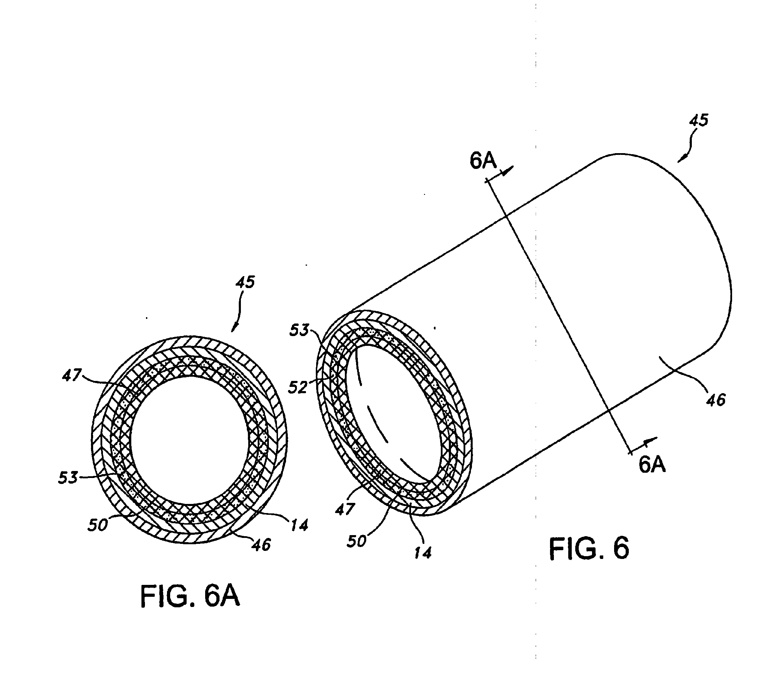

In further aspects of the invention, the implantable structure may be used in conjunction with support structures such as radially-expandable members, stents and other structures which are capable of maintaining patency of the implantable structure in a bodily vessel. For example, a layer of PTFE may be disposed over a support structure and the layer of PTFE being joined to the textile tubular structure via the elastomeric bonding agent, as shown in Figures 5 and 5A; or a support structure may be disposed between a layer of PTFE and a textile layer with the inner PTFE layer being joined to the outer textile layer via the elastomeric bonding agent, as shown in Figures 6 and 6A. Additionally contemplated is the layers being positioned in various combinations. For example, the textile layer being interior to the support structure and PTFE layer being exterior, or textile layer may be disposed over PTFE layer which may be disposed over interior and/or exterior surfaces of stent (not shown).

Any support structure construction known to those skilled in the art may be used. Such support structures typically come in the form of stents, which are formed of metal or polymeric materials generally formed in a tubular structure and are used to hold a vein or artery open. Stents are well known in the art and may be self-expanding or radially expandable by balloon expansion. It is advantageous to use stent/graft configurations because the stent provides and ensures the patency of the prosthesis, while the vascular graft provides biocompatible properties in a vessel more suitable for blood to flow through.

With reference to Figures 5, 5A and Figures 6, 6A, hybrid prostheses **35** and **45** are similar to prostheses **5** or **25,** above-described, being a multi-layered composite graft including PTFE, textile and elastomeric bonding agent having radiopaque material therein. However, hybrid prostheses **35** and **45** further include support structures **4** and **14,** respectively.

With reference to Figure 5 and 5A, a hybrid prosthesis **35** is shown having a tubular support structure 4 interior to a hybrid graft **38.** The hybrid graft **38** being a PTFE layer **37** and a textile layer **36** joined by elastomeric bonding agent **40.** The elastomeric bonding agent **40** having radiopaque material **43** impregnated in an elastomeric matrix **42** in sufficient amounts to produce a radiopaque image. The arrangement of layers may be reversed such that the PTFE layer becomes the external surface of the prosthesis and the textile layer is adjacent to the support structure and the bonding agent is between the textile and PTFE layers (not shown). The hybrid graft **38** may be frictionally secured to the support structure or additional attachment may be desired. Figure 5 shows the ends of the support structure **4** being additionally secured to the hybrid graft **38** by sutures **39.** The hybrid graft **38** is formed to allow for simultaneous radial expansion of the support structure 4 along with the PTFE layer **37** and the textile layer **36.** The radial expansion is preferably unhindered by any of the constituent elements of the hybrid graft **38.**

The support structure **4** may be any structure known in the art which is capable of maintaining patency of the hybrid prosthesis **35** in a bodily vessel. For example, the support structure **4** may be a stent, and preferably is radially-expandable. Radially-expandable member may be of any stent configuration known to those skilled in the art, including those used alone or in a stent/graft arrangement. Various stent types and stent constructions may be employed in the present invention including, without limitation, self-expanding stents and balloon expandable stents. The stents may be capable of radially contracting as well. Self-expanding stents include those that have a spring-like action which cause the stent to radially expand or stents which expand due to the memory properties of the stent material for a particular configuration at a certain temperature. Nitinol^{®} is an example of a material which may be used as a self-expanding stent. Other materials are of course contemplated, such as stainless steel, platinum, gold, titanium, tantalum, niobium, and other biocompatible materials, as well as polymeric stents. The configuration of the stent may also be chosen from a host of geometries. For example, wire stents can be fastened in a continuous helical pattern, with or without wave-like forms or zigzags in the wire, to form a radially deformable stent. Individual rings or circular members can be linked together such as by struts, sutures, or interlacing or locking of the rings to form a tubular stent. Additionally, the configuration of the support structure includes various constructions as known in the art such as a knitted structure, a woven structure, a braided structure, a non-woven spun structure and combinations thereof. Although a wide variety of distensible members may be used, Figure 5 shows one particular support structure 4, a stent, which may be employed in prosthesis 35. The particular stent 4 shown in Figures 5 and 5A is a braided stent.

Braided stents are known in the art, examples of braided stents include but are not limited to those described in U.S. Patent No. 4,655,771 to Hans I. Wallsten, U.S. Patent No. 5,575,818 to Pinchuk, U.S. Patent No. 6,083,257 to Taylor, et al., and U.S. Patent No. 6,622,604 to Chouinard, et al.

Braided stents tend to be very flexible, having the ability to be placed in tortuous anatomy and still maintain patency. The flexibility of braided stents make them particularly well-suited for treating aneurysms in the aorta, where the lumen of the vessel often becomes contorted and irregular both before and after placement of the stent. A typical braided stent includes a first set of filaments wound in a first helical direction (i.e. to the left) and a second set of filaments wound in a second, opposite helical direction (i.e. to the right), forming a plurality of overlaps. The filaments may be wire, such as nitinol or stainless steel, or may comprise polymer or any type of filaments known in the art.

As used herein, a "braided" stent refers to a stent formed of at least two continuous filaments which are interwoven in a pattern, thus forming overlaps. At each overlap, one filament is positioned radially outward relative to the other filament. Following each filament along its helical path through a series of consecutive overlaps, that filament may, for example be in the radial inward position in one overlap and in the radial outward position in a next overlap, or may in the inward position for two overlaps and in the outward position for the next two, and so on. As mentioned above, exemplary braided stents are disclosed in U.S. Pat. No. 4,655,771 to Hans I. Wallsten. A typical braided stent is formed on a mandrel by a braiding or plaiting machine, such as a standard braiding machine known in the art and manufactured by Rotek of Ormond Beach, Fla. Any such braiding or plaiting machine may be used, however, and the use of terminology specific to components of the machine manufactured by Rotek is not intended as a limitation to the use of that machine design. To the extent that the terminology used herein is specific to the components of any one or several machines, it should be understood such components specifically referred to herein generally have corresponding functionally equivalent components with respect to other machines.

With reference to Figures 6 and 6A, an alternative to the hybrid prosthesis is shown therein. A hybrid prosthesis **45** is shown having a tubular support structure **14** interposed between inner PTFE layer **47** and outer textile layer **46.** The layers **46** and **47** are joined using an adhesive bonding agent **50** having radiopaque material **53** incorporated therein. The layers **46, 47** are joined through interstices found in the support structure 14. The ends of the support structure **14** may be affixed to the layers **46, 47,** however depending on the application it may be desirable to have the layers **46, 47** not affixed to the support structure **14.** The layers **46, 47** are thermally bonded with the support structure **14** therebetween and the bonding agent 50 being cured to adhesively secure the hybrid prosthesis. The arrangement of the layers may be altered, wherein the support structure **14** and the PTFE layer **47** may be disposed externally of the textile layer **46** with the layer of bonding agent **50** being interposed between the textile layer **46** and the PTFE layer **47.**

With reference to Figures 5 and 6, the textile layers **36, 46;** the PTFE layers **37, 47;** the bonding agent **40, 50;** the elastomeric matrix **42, 52;** and radiopaque materials **43, 53** may be of any structure described in the embodiments above. Likewise, the interaction between the PTFE layer, the textile layer, and the bonding agent is the same interaction described above. Desirably, the textile structure **36,46** and the PTFE structures **37, 47** are adhesively joined to form a unitary composite.

Referring now to Figures 7 and 7A, a textile reinforced PTFE vascular patch **55** is shown. The vascular patch **55** of the present invention is constructed of a thin layer of PTFE **57** which is generally in an elongate planar shape. The PTFE layer **57** is bonded to a thin layer of textile material **56** which is also formed in an elongate planar configuration. The PTFE layer **57** is bonded to the textile layer **56** by use of an elastomeric bonding agent **60.** The bonding agent **60** includes an elastomeric matrix **62** and a radiopaque material **63** therein in sufficient amounts to provide a radiopaque image. The composite structure **55** can be formed of a thickness less than either conventional textile or PTFE vascular patches. This enables the patch to exhibit enhanced handling characteristics.

As is well known, the vascular patch may be used to seal an incision in the vascular wall or otherwise repair a soft tissue area in the body. The PTFE surface of the vascular patch would be desirably used as the blood contacting side of the patch. This would provide a smooth luminal surface and would reduce thrombus formation. The textile surface is desirably opposed to the blood contacting surface so as to promote cellular ingrowth and healing.

The composite vascular patch may be formed by applying the bonding agent as above described to one surface of the PTFE layer. Thereafter, the textile layer would be applied to the coated layer of PTFE. The composite may be bonded by the application of heat and pressure to form the composite structure. The composite vascular patch of the present invention exhibits many of the above stated benefits of using PTFE in combination with a textile material. The patches of the present invention may also be formed by first making a tubular construction and then cutting the requisite planar shape therefrom.

Various changes to the foregoing described and shown structures will now be evident to those skilled in the art. Accordingly, the particularly disclosed scope of the invention is set forth in the following claims.

## Claims

1. A hybrid implantable vascular prosthesis comprising:
- a PTFE tubular structure (7A, 27A, 37, 47),
**characterized in that** the prosthesis further comprises:
- a textile tubular structure (6A, 26A, 36, 46); and
- a cured elastomeric bonding agent (10A, 30A, 40, 50) adhesively securing said PTFE tubular structure and said textile tubular structure, said elastomeric agent having radiopaque material (13A, 33A, 43, 53) impregnated therein in sufficient amounts to produce a radiopaque image.

2. A hybrid implantable patch comprising:
- a PTFE substantially planar structure (57),
**characterized in that** the patch further comprises:
- a textile substantially planar structure (56);
- a cured elastomeric bonding agent (60) adhesively securing said PTFE substantially planar structure (57) and said textile substantially planar structure (56), said elastomeric agent (60) having radiopaque material (63) impregnated therein in sufficient amounts to produce a radiopaque image.

3. The prosthesis or patch of claim 1 or 2, wherein said radiopaque material (13A, 33A, 43, 53, 63) is metallic particulates.

4. The prosthesis or patch of claim 3, wherein said metallic particulates is selected from a group consisting of gold, barium sulfate and combinations thereof.

5. The prosthesis or patch of any one of claims 1 to 4, wherein said elastomeric bonding agent (10A, 30A, 40, 50, 60) comprises 5 to 40 percent radiopaque material.

6. The prosthesis or patch of any one of claims 1 to 5, wherein said radiopaque material (13A, 33A, 43, 53, 63) comprises particles between the size of about 0.5 and 2.0 microns.

7. The prosthesis or patch of any one of claims 1 to 6, wherein said radiopaque material (13A, 33A, 43, 53, 63) is concentrated in a designated area of the elastomeric bonding agent (10A, 30A, 40, 50, 60).

8. The prosthesis or patch of any one of claims 1 to 6, wherein said radiopaque material (13A, 33A, 43, 53, 63) is uniformly distributed throughout the elastomeric bonding agent (10A, 30A, 40, 50, 60).

9. The prosthesis or patch of any one of claims 1 to 8, wherein said textile structure (6A, 26A, 36, 46, 56) is a knitted textile structure, a woven textile structure, a braided textile structure, a non-woven spun structure and combinations thereof.

10. The prosthesis or patch of any one of claims 1 to 9, wherein said PTFE structure (7A, 27A, 37, 47, 57) is non-porous structure.

11. The prosthesis or patch of any one of claims 1 to 9, wherein said PTFE structure (7A, 27A, 37, 47, 57) is porous structure.

12. The prosthesis or patch of any one of claims 1 to 11, wherein said elastomeric bonding agent (10A, 30A, 40, 50, 60) is selected from the group consisting of polyurethanes, styrene/isobutylene/styrene block copolymers, silicones, and combinations thereof.

13. The prosthesis of any one of claims 1 and 3 to 12, further comprising a support tubular structure (4, 14), wherein the cured elastomeric bonding agent (40, 50) adhesively secures said PTFE structure (37, 47), said textile structure (36, 46) and said support structure (4, 14).

14. The prosthesis of claim 13, wherein said support structure (4, 14) is a knitted structure, a woven structure, a braided structure, a non-woven spun structure and combinations thereof.

15. The prosthesis of claim 1, further comprising a braided stent frame, wherein the PTFE structure is a nonporous PTFE layer covering said stent frame, wherein the textile structure is a polyester knitted textile layer covering said PTFE layer; and wherein the cured elastomeric bonding agent is a cured radiopaque elastomeric composition thermally bonding said PTFE layer and said textile layer, said radiopaque elastomeric composition comprising radiopaque metallic particulates and a polyurethane adhesive, said polyurethane adhesive being impregnated with said particulates, in sufficient amounts to produce a radiopaque image.

16. The prosthesis of claim 15, wherein said radiopaque metallic particulates is selected from a group consisting of gold, barium sulfate and combinations thereof.

17. A method of forming a hybrid device comprising the steps of:
(a) providing a PTFE layer having opposed surfaces;
(b) providing a textile layer having opposed surfaces;
(c) providing a radiopaque elastomeric composition comprising an elastomeric bonding agent, and radiopaque metallic particulates, said particulates being impregnated into said elastomeric agent in sufficient amounts to produce a radiopaque image upon implantation of said device.
(d) applying a layer of said radiopaque elastomeric composition to one of said opposed surfaces of said PTFE layer or said textile layer;
(e) placing the other of said PTFE layer or said textile layer on top of said layer of radiopaque elastomeric composition, thereby defining a hybrid assembly having an interior surface and an exterior surface wherein said interior surface of said hybrid is one of said PTFE layer or said textile layer, and said exterior surface of said hybrid is the other of said PTFE layer or said textile layer;
(f) applying heat to said radiopaque elastomeric composition to adhesively bond said textile layer and said PTFE layer; and
(g) encapsulating said radiopaque material between said textile layer and said PTFE layer to provide a laminated hybrid assembly.

18. The method of claim 17, wherein said PTFE is nonporous.

19. The method of claim 17, wherein said PTFE is porous with a microporous structure of nodes interconnected by fibrils.

20. The method of claim 19, wherein the step of applying said layer of said radiopaque elastomeric composition includes applying said layer to one of said opposed surfaces of said PTFE layer with said radiopaque elastomeric composition being disposed within said microporous structure.

21. The method of any one of claims 17 to 20, wherein said textile layer is a hollow tubular textile layer having an inner and outer textile surface and said PTFE layer is applied to said inner textile surface.

22. The method of any one of claims 17 to 21, wherein said textile layer is a hollow tubular textile layer having an inner and outer textile surface and said PTFE layer is applied to said outer textile surface.

23. The method of any one of claims 17 to 22, wherein the step of applying heat to said radiopaque elastomeric composition includes heating said composition from 163°C-232°C (325°F. to 450°F).

24. The method of any one of claims 17 to 23, further including the steps of:
providing a support structure; and
placing said support structure between said textile layer and said PTFE layer.

25. The method of claim 24, wherein said support structure is a knitted stent, a woven stent, a braided stent, a non-woven spun structure and combinations thereof.

26. The method of any one of claims 17 to 23, further including the steps of:
providing a support structure; and
placing said PTFE layer and said textile layer onto said support structure.

27. The method of claim 24 or 26, wherein said support structure is a stent, said stent having two opposed ends and a stent wall therebetween.

28. The method of claim 27, wherein said stent is a knitted stent, a woven stent, a stretchknit stent, a braided stent, and combinations thereof.

29. The method of claim 27, further including the steps of:
suturing said hybrid assembly to said stent at said opposed ends of said stent.

30. The method of any one of claims 17 to 29, wherein said elastomeric bonding agent is selected from the group consisting of polyurethanes, styrene/isobutylene/styrene block copolymers, silicones, and combinations thereof.

31. The method of any one of claims 17 to 30, wherein said elastomeric bonding agent is a polycarbonate urethane.

32. The method of any one of claims 17 to 31, wherein said textile layer is knitted textile layer, a woven textile layer, a braided textile layer, a non-woven spun textile layer and combinations thereof.

33. The method of any one of claims 17 to 20 and 30 to 32, wherein said textile layer and said PTFE layer are substantially planar.

34. The method of claim 33, wherein said hybrid device is a vascular patch.

## Patentansprüche

1. Implantierbare Gefäßhybridprothese, umfassend:
- eine rohrförmige PTFE-Struktur (7A, 27A, 37, 47),
**dadurch gekennzeichnet, daß** die Prothese ferner umfaßt:
- eine rohrförmige Textilstruktur (6A, 26A, 36, 46) und
- ein gehärtetes elastomeres Bindemittel (10A, 30A, 40, 50), welches die rohrförmige PTFE-Struktur und die rohrförmige Textilstruktur adhäsiv verbindet, wobei in das elastomere Mittel röntgendichtes Material (13A, 33A, 43, 53) in einer Menge eingebettet ist, die ausreicht, um ein röntgendichtes Bild zu erzeugen.

2. Implantierbarer Hybridpatch umfassend:
- eine im wesentlichen planare PTFE-Struktur (57),
**dadurch gekennzeichnet, daß** der Patch ferner umfaßt:
- eine im wesentlichen planare Textilstruktur (56) und
- ein gehärtetes elastomeres Bindemittel (60), welches die im wesentlichen planare PTFE-Struktur (57) und die im wesentlichen planare Textilstruktur (56) adhäsiv verbindet, wobei in das elastomere Mittel (60) röntgendichtes Material (63) in einer Menge eingebettet ist, die ausreicht, um ein röntgendichtes Bild zu erzeugen.

3. Prothese oder Patch nach Anspruch 1 oder 2, wobei das röntgendichte Material (13A, 33A, 43, 53, 63) aus Metallpartikeln besteht.

4. Prothese oder Patch nach Anspruch 3, wobei die Metallpartikel aus einer aus Gold, Bariumsulfat und Kombinationen davon bestehenden Gruppe ausgewählt sind.

5. Prothese oder Patch nach einem der Ansprüche 1 bis 4, wobei das elastomere Bindemittel (10A, 30A, 40, 50, 60) 5 bis 40 Prozent röntgendichtes Material umfaßt.

6. Prothese oder Patch nach einem der Ansprüche 1 bis 5, wobei das röntgendichte Material (13A, 33A, 43, 53, 63) Partikel der Größe zwischen ca. 0,5 und 2,0 Mikron umfaßt.

7. Prothese oder Patch nach einem der Ansprüche 1 bis 6, wobei das röntgendichte Material (13A, 33A, 43, 53, 63) in einem bestimmten Bereich des elastomeren Bindemittels (10A, 30A, 40, 50, 60) konzentriert ist.

8. Prothese oder Patch nach einem der Ansprüche 1 bis 6, wobei das röntgendichte Material (13A, 33A, 43, 53, 63) über das ganze elastomere Bindemittel (10A, 30A, 40, 50, 60) gleichmäßig verteilt ist.

9. Prothese oder Patch nach einem der Ansprüche 1 bis 8, wobei die Textilstruktur (6A, 26A, 36, 46, 56) eine gestrickte Textilstruktur, eine gewebte Textilstruktur, eine geflochtene Textilstruktur, eine Spinnvliesstruktur und Kombinationen davon ist.

10. Prothese oder Patch nach einem der Ansprüche 1 bis 9, wobei die PTFE-Struktur (7A, 27A, 37, 47, 57) eine nicht poröse Struktur ist.

11. Prothese oder Patch nach einem der Ansprüche 1 bis 9, wobei die PTFE-Struktur (7A, 27A, 37, 47, 57) eine poröse Struktur ist.

12. Prothese oder Patch nach einem der Ansprüche 1 bis 11, wobei das elastomere Bindemittel (10A, 30A, 40, 50, 60) aus der aus Polyurethanen, Styrol/Isobutylen/Styrol-Block-Copolymeren, Silikonen und Kombinationen davon bestehenden Gruppe ausgewählt ist.

13. Prothese nach einem der Ansprüche 1 und 3 bis 12, ferner umfassend eine rohrförmige Stützstruktur (4, 14), wobei das gehärtete elastomere Bindemittel (40, 50) die PTFE-Struktur (37, 47), die Textilstruktur (36, 46) und die Stützstruktur (4, 14) adhäsiv verbindet.

14. Prothese nach Anspruch 13, wobei die Stützstruktur (4, 14) eine gestrickte Struktur, eine gewebte Struktur, eine geflochtene Struktur, eine Spinnvliesstruktur und Kombinationen davon ist.

15. Prothese nach Anspruch 1, ferner umfassend einen geflochtenen Stentrahmen, wobei die PTFE-Struktur eine nicht poröse PTFE-Schicht ist, die den Stentrahmen abdeckt, wobei die Textilstruktur eine gestrickte Polyester-Textilschicht ist, die die PTFE-Schicht abdeckt, und wobei das gehärtete elastomere Bindemittel eine gehärtete röntgendichte Elastomerzusammensetzung ist, die die PTFE-Schicht und die Textilschicht thermisch verbindet, wobei die röntgendichte Elastomerzusammensetzung röntgendichte Metallpartikel und einen Polyurethan-Klebstoff umfaßt, wobei der Polyurethan-Klebstoff mit den Partikeln in einer Menge versehen ist, die ausreicht, ein röntgendichtes Bild zu erzeugen.

16. Prothese nach Anspruch 15, wobei die röntgendichten Metallpartikel aus der aus Gold, Bariumsulfat und Kombinationen davon bestehenden Gruppe ausgewählt sind.

17. Verfahren zur Herstellung einer Hybridvorrichtung, umfassend die Schritte:
(a) Bereitstellen einer PTFE-Schicht mit gegenüberliegenden Flächen,
(b) Bereitstellen einer Textilschicht mit gegenüberliegenden Flächen,
(c) Bereitstellen einer röntgendichten Elastomerzusammensetzung umfassend ein elastomeres Bindemittel und röntgendichte Metallpartikel, wobei die Partikel in dem elastomeren Mittel in einer Menge einbettet werden, die ausreicht, nach Implantation der Vorrichtung ein röntgendichtes Bild zu erzeugen,
(d) Auftragen einer Schicht der röntgendichten Elastomerzusammensetzung auf eine der gegenüberliegenden Flächen der PTFE-Schicht oder der Textilschicht,
(e) Plazieren der anderen von der PTFE-Schicht oder der Textilschicht auf der Schicht aus röntgendichter Elastomerzusammensetzung, wodurch eine Hybridanordnung mit einer inneren Fläche und einer äußeren Fläche definiert wird, wobei die innere Fläche des Hybrids eine von der PTFE-Schicht oder der Textilschicht ist und die äußere Fläche des Hybrids die andere von der PTFE-Schicht oder der Textilschicht ist,
(f) Zuführen von Wärme zu der röntgendichten Elastomerzusammensetzung, um die Textilschicht und die PTFE-Schicht adhäsiv zu verbinde, und
(g) Einkapseln des röntgendichten Materials zwischen der Textilschicht und der PTFE-Schicht, um eine laminierte Hybridanordnung bereitzustellen.

18. Verfahren nach Anspruch 17, wobei das PTFE nicht porös ist.

19. Verfahren nach Anspruch 17, wobei das PTFE porös ist und eine mikroporöse Struktur aus Knoten aufweist, die durch Fibrillen miteinander verbunden sind.

20. Verfahren nach Anspruch 19, wobei der Schritt des Auftragens der Schicht der röntgendichten Elastomerzusammensetzung das Auftragen der Schicht auf eine der gegenüberliegenden Flächen der PTFE-Schicht umfaßt, wobei die röntgendichte Elastomerzusammensetzung innerhalb der mikroporösen Struktur angeordnet wird.

21. Verfahren nach einem der Ansprüche 17 bis 20, wobei die Textilschicht eine hohle rohrförmige Textilschicht mit einer inneren und äußeren Textilfläche ist und die PTFE-Schicht auf die innere Textilfläche aufgetragen wird.

22. Verfahren nach einem der Ansprüche 17 bis 21, wobei die Textilschicht eine hohle rohrförmige Textilschicht mit einer inneren und äußeren Textilfläche ist und die PTFE-Schicht auf die äußere Textilfläche aufgetragen wird.

23. Verfahren nach einem der Ansprüche 17 bis 22, wobei der Schritt des Zuführens von Wärme zu der röntgendichten Elastomerzusammensetzung das Erwärmen der Zusammensetzung von 163°C - 232°C (325°F bis 450°F) umfaßt.

24. Verfahren nach einem der Ansprüche 17 bis 23, ferner umfassend die Schritte des:
Bereitstellens einer Stützstruktur und
Plazierens der Stützstruktur zwischen der Textilschicht und der PTFE-Schicht.

25. Verfahren nach Anspruch 24, wobei die Stützstruktur ein gestrickter Stent, ein gewebter Stent, ein geflochtener Stent, eine Spinnvliesstruktur und Kombinationen davon ist.

26. Verfahren nach einem der Ansprüche 17 bis 23, ferner umfassend die Schritte des:
Bereitstellens einer Stützstruktur, und
Plazierens der PTFE-Schicht und der Textilschicht auf der Stützstruktur.

27. Verfahren nach Anspruch 24 oder 26, wobei die Stützstruktur ein Stent ist, wobei der Stent zwei sich gegenüberliegende Enden und dazwischen eine Stentwand aufweist.

28. Verfahren nach Anspruch 27, wobei der Stent ein gestrickter Stent, ein gewebter Stent, ein stretch-gestrickter Stent, ein geflochtener Stent und Kombinationen davon ist.

29. Verfahren nach Anspruch 27, ferner umfassend den Schritte des Vernähens der Hybridanordnung an dem Stent an den sich gegenüberliegenden Enden des Stents.

30. Verfahren nach einem der Ansprüche 17 bis 29, wobei das elastomere Bindemittel aus der aus Polyurethanen, Styrol/Isobutylen/Styrol-Block Copolymeren, Silikonen und Kombinationen davon bestehenden Gruppe ausgewählt wird.

31. Verfahren nach einem der Ansprüche 17 bis 30, wobei das elastomere Bindemittel ein Polycarbonaturethan ist.

32. Verfahren nach einem der Ansprüche 17 bis 31, wobei die Textilschicht eine gestrickte Textilschicht, eine gewebte Textilschicht, eine geflochtene Textilschicht, eine Spinnvliestextilschicht und Kombinationen davon ist.

33. Verfahren nach einem der Ansprüche 17 bis 20 und 30 bis 32, wobei die Textilschicht und die PTFE-Schicht im wesentlichen planar sind.

34. Verfahren nach Anspruch 33, wobei die Hybridvorrichtung ein Gefäßpatch ist.

## Revendications

1. Prothèse vasculaire implantable hybride comprenant :
- une structure tubulaire en PTFE (7A, 27A, 37, 47),
**caractérisée en ce que** la prothèse comprend en outre :
- une structure tubulaire textile (6A, 26A, 36, 46) ; et
- un agent liant élastomérique durci (10A, 30A, 40, 50) fixant de manière adhésive ladite structure tubulaire en PTFE et ladite structure tubulaire textile, ledit agent élastomérique ayant une matière radio-opaque (13A, 33A, 43, 53) imprégnée en quantités suffisantes pour produire une image radio-opaque.

2. Plaque implantable hybride comprenant :
- une structure sensiblement plane en PTFE (57),
**caractérisée en ce que** la plaque comprend en outre :
- une structure textile sensiblement plane (56) ;
- un agent liant élastomérique durci (60) fixant de manière adhésive ladite structure sensiblement plane en PTFE (57) et ladite structure textile sensiblement plane (56), ledit agent élastomérique (60) ayant une matière radio-opaque (63) imprégnée en quantités suffisantes pour produire une image radio-opaque.

3. Prothèse ou plaque selon la revendication 1 ou 2, dans laquelle ladite matière radio-opaque (13A, 33A, 43, 53, 63) est une matière particulaire métallique.

4. Prothèse ou plaque selon la revendication 3, dans laquelle ladite matière particulaire métallique est choisie dans un groupe consistant en l'or, le sulfate de baryum et leurs combinaisons.

5. Prothèse ou plaque selon l'une quelconque des revendications 1 à 4, dans laquelle ledit agent liant élastomérique (10A, 30A, 40, 50, 60) comprend 5 % à 40% de matière radio-opaque.

6. Prothèse ou plaque selon l'une quelconque des revendications 1 à 5, dans laquelle ladite matière radio-opaque (13A, 33A, 43, 53, 63) comprend des particules entre la taille d'environ 0,5 et 2,0 µm.

7. Prothèse ou plaque selon l'une quelconque des revendications 1 à 6, dans laquelle ladite matière radio-opaque (13A, 33A, 43, 53, 63) est concentrée dans une zone déterminée de l'agent liant élastomérique (10A, 30A, 40, 50, 60).

8. Prothèse ou plaque selon l'une quelconque des revendications 1 à 6, dans laquelle ladite matière radio-opaque (13A, 33A, 43, 53, 63) est distribuée uniformément dans tout l'agent liant élastomérique (10A, 30A, 40, 50, 60).

9. Prothèse ou plaque selon l'une quelconque des revendications 1 à 8, dans laquelle ladite structure textile (6A, 26A, 36, 46, 56) est une structure textile tricotée, une structure textile tissée, une structure textile tressée, une structure filée non tissée et leurs combinaisons.

10. Prothèse ou plaque selon l'une quelconque des revendications 1 à 9, dans laquelle ladite structure en PTFE (7A, 27A, 37, 47, 57) est une structure non poreuse.

11. Prothèse ou plaque selon l'une quelconque des revendications 1 à 9, dans laquelle ladite structure en PTFE (7A, 27A, 37, 47, 57) est une structure poreuse.

12. Prothèse ou plaque selon l'une quelconque des revendications 1 à 11, dans laquelle ledit agent liant élastomérique (10A, 30A, 40, 50, 60) est choisi dans le groupe consistant en les polyuréthanes, les copolymères bloc styrène/isobutylène/styrène, les silicones et leurs combinaisons.

13. Prothèse selon l'une quelconque des revendications 1 et 3 à 12 comprenant en outre une structure tubulaire de support (4, 14) dans laquelle l'agent liant élastomérique durci (40, 50) fixe de manière adhésive ladite structure en PTFE (37, 47), ladite structure textile (36, 46) et ladite structure de support (4, 14).

14. Prothèse selon la revendication 13, dans laquelle ladite structure de support (4, 14) est une structure tricotée, une structure tissée, une structure tressée, une structure filée non tissée et leurs combinaisons.

15. Prothèse selon la revendication 1 comprenant en outre une ossature de stent tressée, dans laquelle la structure en PTFE est une couche de PTFE non poreuse recouvrant ladite ossature de stent, dans laquelle la structure textile est une couche textile tricotée en polyester recouvrant ladite couche de PTFE ; et où l'agent liant élastomérique durci est une composition élastomérique radio-opaque durcie liant thermiquement ladite couche de PTFE et ladite couche textile, ladite composition élastomérique radio-opaque comprenant des particules métalliques radio-opaques et un adhésif polyuréthane, ledit adhésif polyuréthane étant imprégné desdites particules, en quantités suffisantes pour produire une image radio-opaque.

16. Prothèse selon la revendication 15, dans laquelle lesdites particules métalliques radio-opaques sont choisies dans un groupe consistant en l'or, le sulfate de baryum et leurs combinaisons.

17. Procédé de formation d'un dispositif hybride comprenant les étapes de :
(a) fourniture d'une couche de PTFE ayant des surfaces opposées ;
(b) fourniture d'une couche textile ayant des surfaces opposées ;
(c) fourniture d'une composition élastomérique radio-opaque comprenant un agent liant élastomérique et des particules métalliques radio-opaques, lesdites particules étant imprégnées dans ledit agent élastomérique en quantités suffisantes pour produire une image radio-opaque lors de l'implantation dudit dispositif
(d) application d'une couche de ladite composition élastomérique radio-opaque à l'une desdites surfaces opposées de ladite couche de PTFE ou de ladite couche textile ;
(e) mise en place de l'autre de ladite couche de PTFE ou de ladite couche textile sur ladite couche de composition élastomérique radio-opaque, pour définir ainsi un ensemble hybride ayant une surface intérieure et une surface extérieure, dans lequel ladite surface intérieure dudit hybride est l'une de ladite couche de PTFE ou de ladite couche textile, et ladite surface extérieure dudit hybride est l'autre de ladite couche de PTFE ou de ladite couche textile ;
(f) application de chaleur à ladite composition élastomérique radio-opaque pour lier de manière adhésive ladite couche textile et ladite couche de PTFE ; et
(g) encapsulation de ladite matière radio-opaque entre ladite couche textile et ladite couche de PTFE pour fournir un ensemble hybride stratifié.

18. Procédé selon la revendication 17 dans lequel ledit PTFE est non poreux.

19. Procédé selon la revendication 17, dans lequel ledit PTFE est poreux avec une structure microporeuse de noeuds reliés entre eux par des fibrilles.

20. Procédé selon la revendication 19, dans lequel l'étape d'application de ladite couche de ladite composition élastomérique radio-opaque inclut l'application de ladite couche à l'une desdites surfaces opposées de ladite couche de PTFE, ladite composition élastomérique radio-opaque étant disposée dans ladite structure microporeuse.

21. Procédé selon l'une quelconque des revendications 17 à 20, dans lequel ladite couche textile est une couche textile tubulaire creuse ayant une surface textile interne et une surface textile externe et ladite couche de PTFE est appliquée à ladite surface textile interne.

22. Procédé selon l'une quelconque des revendications 17 à 21, dans lequel ladite couche textile est une couche textile tubulaire creuse ayant une surface textile interne et une surface textile externe et ladite couche de PTFE est appliquée à ladite surface textile externe.

23. Procédé selon l'une quelconque des revendications 17 à 22, dans lequel l'étape d'application de chaleur à ladite composition élastomérique radio-opaque inclut le chauffage de ladite composition entre 163°C-232°C (325°F à 450°F).

24. Procédé selon l'une quelconque des revendications 17 à 23 incluant en outre les étapes de :
fourniture d'une structure de support ; et
mise en place de ladite structure de support entre ladite couche textile et ladite couche de PTFE.

25. Procédé selon la revendication 24, dans lequel ladite structure de support est un stent tricoté, un stent tissé, un stent tressé, une structure filée non tissée et leurs combinaisons.

26. Procédé selon l'une quelconque des revendications 17 à 23 incluant en outre les étapes de :
fourniture d'une structure de support ; et
mise en place de ladite couche de PTFE et de ladite couche textile sur ladite structure de support.

27. Procédé selon la revendication 24 ou 26, dans lequel ladite structure de support est un stent, ledit stent ayant deux extrémités opposées et une paroi de stent entre elles.

28. Procédé selon la revendication 27, dans lequel ledit stent est un stent tricoté, un stent tissé, un stent tricoté et étiré, un stent tressé et leurs combinaisons.

29. Procédé selon la revendication 27 incluant en outre les étapes de :
suture dudit ensemble hybride audit stent au niveau desdites extrémités opposées dudit stent.

30. Procédé selon l'une quelconque des revendications 17 à 29, dans lequel ledit agent liant élastomérique est choisi dans le groupe consistant en les polyuréthanes, les copolymères bloc styrène/isobutylène/ styrène, les silicones et leurs combinaisons.

31. Procédé selon l'une quelconque des revendications 17 à 30, dans lequel ledit agent liant élastomérique est un polycarbonate uréthane.

32. Procédé selon l'une quelconque des revendications 17 à 31, dans lequel ladite couche textile est une couche textile tricotée, une couche textile tissée, une couche textile tressée, une couche textile filée non tissée et leurs combinaisons.

33. Procédé selon l'une quelconque des revendications 17 à 20 et 30 à 32, dans lequel ladite couche textile et ladite couche de PTFE sont sensiblement planes.

34. Procédé selon la revendication 33, dans lequel ledit dispositif hybride est une plaque vasculaire.
